# EUROPEAN PATENT APPLICATION

(11) **EP 4 767 953 A1**
(43) Date of publication of application: **01.07.2026**
(21) Application number: 24855755.5
(22) Date of filing: 18.08.2024
(51) Int. Cl.: A61B 17/32, A61M 25/10, A61B 90/00, G06N 20/00

(54) **AUTOMATIC DETERMINATION METHOD AND SYSTEM FOR INTRAVASCULAR NERVE ABLATION END POINT**

(30) Priority: 23.08.2023 CN 202311067936
(71) Applicant: Brattea Medtech Co., Ltd., Shanghai 201114 (CN)
(72) Inventor: GUO, Jiulin, Shanghai 200231 (CN); HUANG, Hanli, Shanghai 200231 (CN)
(74) Representative: Wang, Bo
(86) International application number: PCT/CN2024/112940
(87) International publication number: WO 2025/040032

(57) **Abstract**

An automatic determination method and system for an intravascular nerve ablation end point. The method comprises the following steps: setting working parameters; injecting normal saline into a balloon when moving the balloon to a target ablation area; measuring the pressure in the balloon and the impedance of the outer surface of the balloon and feeding back the pressure and the impedance to a control system; starting an ablation mode by an ultrasonic transducer, and starting to calculate an ablation duration; the ultrasonic transducer switching to a temperature measurement mode, measuring the temperature of the ablation area and feeding back the temperature to the control system; and on the basis of related working parameters measured in real time, the control system determining, by means of a machine learning model, whether an ablation end point of the area is reached. According to the method and the system, by setting a plurality of working parameters and performing measurement in real time, and by means of intelligent evaluation by the machine learning model, high-precision automatic determination for an intravascular nerve ablation end point is achieved.

## Description

### BACKGROUND

### Technical Field

The present disclosure relates to an automatic determination method for an intravascular nerve ablation end point, and relates to a corresponding automatic determination system for an intravascular nerve ablation end point, belonging to the technical field of medical devices.

### Related Art

An intravascular ultrasonic ablation technology is an interventional surgical method. By the method, an ultrasonic transducer is sent to a specific position in a body, and lesion tissue in a target area is ablated by using thermal energy generated by ultrasonic waves. However, most of the existing ultrasonic ablation apparatuses rely on manual control, or determine an ablation process only according to a single parameter, and lack a real-time feedback mechanism. This causes an inappropriate ultrasonic ablation time to affect a treatment effect or damaging surrounding normal tissue. To effectively improve accuracy and safety of ultrasonic ablations, researchers are actively exploring automatic determination methods for an intravascular nerve ablation end point.

### SUMMARY

A first technical problem to be resolved by the present disclosure is to provide an automatic determination method for an intravascular nerve ablation end point.

Another technical problem to be resolved by the present disclosure is to provide an automatic determination method for an intravascular nerve ablation end point.

To achieve the above objectives, the present disclosure adopts the following technical solutions:
According to a first aspect of the embodiments of the present disclosure, an automatic determination method for an intravascular nerve ablation end point is provided. The method includes the following steps:
(1) setting working parameters, where the working parameters at least include an ablation area, an ultrasonic working frequency, an ultrasonic output power, a pressure inside a balloon, an ablation duration, and an ablation area temperature;
(2) moving the balloon with a built-in ultrasonic transducer to a specified position in a target ablation area;
(3) injecting normal saline into the balloon, and maintaining consistent outflow and inflow velocities until the pressure inside the balloon reaches a set target value;
(4) detecting the pressure inside the balloon and an outer surface impedance of the balloon in real time, and feeding back the pressure and the outer surface impedance to a control system;
(5) enabling, by the ultrasonic transducer, an ablation mode to start ablation work, and starting, by the control system, to calculate the ablation duration;
(6) switching the ultrasonic transducer to a temperature measurement mode to detect the ablation area temperature, and feeding back the temperature to the control system; starting, by the control system, to calculate a sustained duration in a case that an ablation area temperature set value is reached;
(7) determining, by the control system according to related working parameters detected in real time, whether an end point of an ablation in the area is reached; performing step (9) in a case that the end point of the ablation in the area is reached; performing step (8) in a case that the end point of the ablation in the area is not reached;
(8) performing step (5) after the control system adjusts a value of the ultrasonic output power according to the ablation area temperature and the related working parameters detected in real time, and repeatedly performing steps (5) to (8);
(9) determining whether current ablation work is completed; performing step (10) in a case that the current ablation work is completed; performing step (2), and repeatedly performing steps (2) to (9) in a case that the current ablation work is not completed; and
(10) ending.

Preferably, working modes of the ultrasonic transducer include an ablation mode and a temperature measurement mode, where the ultrasonic transducer outputs relatively high power when working in the ablation mode, and outputs relatively low power when working in the temperature measurement mode.

Preferably, the end point of the ablation in the area is determined by using a machine learning model, and the machine learning model automatically performs ablation effect evaluation and end point determination according to the related working parameters input by the control system, where determination of reaching the end point of the ablation include two cases: an ablation effect reaches an expected target and an abnormal interruption occurs.

Preferably, the related working parameters input by the machine learning model at least include set values, measured values, and variations of the ultrasonic output power, the ablation duration, the sustained duration, the ablation area temperature, the pressure inside the balloon, and the outer surface impedance of the balloon.

Preferably, in an ablation working process, the machine learning model determines that the abnormal interruption occurs and stops ablation work in a current ablation area to reach the end point of the ablation when at least one of the following cases occurs: the ablation duration exceeds a set value, a change of the outer surface impedance of the balloon abruptly changes abnormally, or a value of the pressure inside the balloon exceeds a set value and abruptly changes abnormally.

Preferably, in an ablation working process, the control system adjusts a value of output power of the ultrasonic transducer in a range below a set value of the ultrasonic output power according to the ablation area temperature and the related working parameters detected in real time, to achieve a target ablation effect.

According to a second aspect of the embodiments of the present disclosure, an automatic determination method for an intravascular nerve ablation end point is provided, including an ablation control module, a parameter detection module, an ultrasonic transducer module, and an end point determination module.

The ablation control module is configured to: configure working parameters for starting ablation work, where the working parameters at least include an ablation area, an ultrasonic working frequency, an ultrasonic output power, a pressure inside a balloon, an ablation duration, and an ablation area temperature; and perform comprehensive monitoring on an ablation working process.

The parameter detection module is configured to detect actual values of related working parameters in real time in the ablation working process, and feed back the actual values to the ablation control module and the end point determination module, where the related working parameters at least include the pressure inside the balloon, an outer surface impedance of the balloon, and the ablation area temperature.

The ultrasonic transducer module is configured to: output ultrasonic energy to perform ablation treatment on nerve tissue, where working modes of the ultrasonic transducer module include an ablation mode and a temperature measurement mode; and adjust the ultrasonic output power according to the ablation area temperature and the related working parameters detected in real time in the ablation working process, to achieve a target ablation effect.

The end point determination module is based on a machine learning model, and is configured to automatically evaluate and determine an end point of an ablation according to a plurality of input working parameters and variations of the plurality of input working parameters.

Preferably, the end point determination module determines that an abnormal interruption occurs and stops ablation work in a current ablation area to reach the end point of the ablation when at least one of the following cases occurs: the ablation duration exceeds a set value, a change of the outer surface impedance of the balloon abruptly changes abnormally, or a value of the pressure inside the balloon exceeds a set value and abruptly changes abnormally.

Compared with an existing technology, according to the automatic determination method for an intravascular nerve ablation end point provided in the embodiments of the present disclosure, high-accuracy automatic determination of the end point of the intravascular nerve ablation is achieved by setting and detecting a plurality of working parameters in real time and combining intelligent evaluation of the machine learning model. The method effectively avoids a false determination caused by determining relying on a single parameter, ensures safety in an ablation process, and optimizes an ablation effect. In addition, the method can adjust the ultrasonic output power according to real-time feedback, achieve the target ablation effect, and record data of each ablation to iteratively optimize the machine learning model, thereby continuously improving accuracy of determining the end point of the intravascular nerve ablation.

### BRIEF DESCRIPTION OF THE DRAWINGS

FIG. 1 is a flowchart of an automatic determination method for an intravascular nerve ablation end point according to an embodiment of the present disclosure;
FIG. 2 is a block diagram of related working parameters input for single determination of an end point of an ablation according to an embodiment of the present disclosure;
FIG. 3 is a schematic diagram of related working parameters for single determination of an end point of an ablation according to an embodiment of the present disclosure; and
FIG. 4 is a block diagram of an automatic determination method for an intravascular nerve ablation end point according to an embodiment of the present disclosure.

### DETAILED DESCRIPTION

The following describes technical content of the present disclosure in detail with reference to accompanying drawings and specific embodiments.

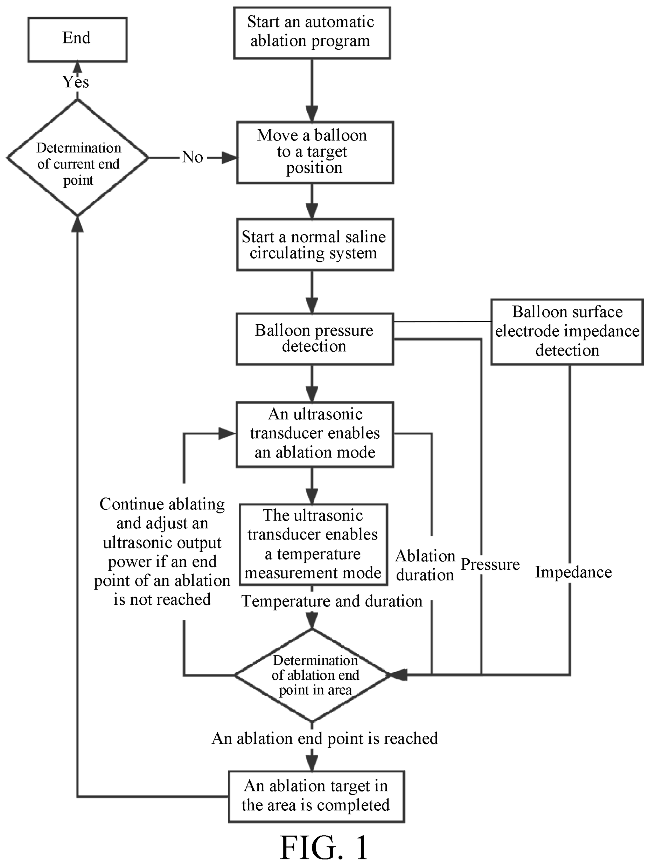

As shown in FIG. 1, an embodiment of the present disclosure provides an automatic determination method for an intravascular nerve ablation end point, including the following steps:
S1: Set working parameters. The working parameters at least include an ablation area, an ultrasonic working frequency, an ultrasonic output power, a pressure inside a balloon, an ablation duration, and an ablation area temperature.
S2: Move the balloon with a built-in ultrasonic transducer to a specified position in a target ablation area.
S3: Inject normal saline into the balloon, and maintain consistent outflow and inflow velocities until the pressure inside the balloon reaches a set target value.
S4: Detect the pressure inside the balloon and an outer surface impedance of the balloon in real time, and feed back the pressure and the outer surface impedance to a control system.
S5: The ultrasonic transducer enables an ablation mode to start ablation work, and the control system starts to calculate the ablation duration.
S6: The ultrasonic transducer switches to a temperature measurement mode to detect the ablation area temperature, and feeds back the temperature to the control system; and the control system starts to calculate a sustained duration in a case that an ablation area temperature set value is reached.
S7: The control system determines, according to related working parameters detected in real time, whether an end point of an ablation in the area is reached. S9 is performed in a case that the end point of the ablation in the area is reached. S8 is performed in a case that the end point of the ablation in the area is not reached.
S8: S5 is performed after the control system adjusts a value of the ultrasonic output power according to the ablation area temperature and the related working parameters detected in real time, and S5 to S8 are repeatedly performed.
S9: Determine whether current ablation work is completed. S10 is performed in a case that the current ablation work is completed; and S2 is performed in a case that the current ablation work is not completed, and S2 to S9 are repeatedly performed.
S10: End.

In the above automatic determination method for an intravascular nerve ablation end point, an involved ultrasonic ablation apparatus is provided with a flexible ultrasonic ablation balloon (briefly referred to as a balloon). The ultrasonic transducer is located inside the balloon, and is configured to output ultrasonic energy to perform ablation on nerve tissue at a lesion. Circulating normal saline injected into the balloon is configured to cool.

In an embodiment of the present disclosure, when the working parameters are set in S1, the working parameters at least include, but are not limited to, an ablation area, an ultrasonic working frequency, an ultrasonic output power, a pressure inside the balloon, an ablation duration, and an ablation area temperature. The ultrasonic working frequency, the ultrasonic output power, and the ablation duration are related to evaluation of actual output ultrasonic energy. The pressure inside the balloon is related to evaluation of an apposition condition between the balloon and a blood vessel wall and a change of a blood vessel morphology in an ablation process. The ablation area temperature is related to evaluation of an actual ablation effect.

According to different ablation areas and different ablation objectives, the control system sets different values for the ultrasonic output power. Generally, according to consideration in two aspects of ablation effect and safety, an ultrasonic wave with relatively low power is required to perform ablation on relatively small nerve tissue, so as to avoid unnecessary damage to surrounding tissue. However, for relatively large nerve tissue, an ultrasonic wave with relatively high power is required to achieve a thorough ablation effect.

It is to be noted that, the set value of the ultrasonic output power is a maximum value. In the ablation process, the control system may adjust the ultrasonic output power in real time within a range defined by the maximum value according to actually fed-back relevant parameters.

For setting the ablation area temperature, a higher temperature value indicates a faster nerve ablation effect. However, in consideration of safety, to avoid damage caused by a high temperature to other tissue, the control system generally sets a temperature value to 60°C. At the ablation temperature, nerve cells are irreversibly damaged only after a period of time.

The control system sets different values for the pressure inside the balloon according to different ablation areas, and a value of the pressure inside the balloon and a variation of the pressure inside the balloon are important parameters for automatically determining an end point of an ablation. Before the ablation starts, when the measured pressure inside the balloon reaches a set pressure value, it is considered that the balloon apposes to the blood vessel wall. In the ablation process, both a diameter of a blood vessel and elasticity of the blood vessel change correspondingly, and for a blood vessel at a determined position, a change range of the blood vessel is correspondingly determined. Therefore, according to different ultrasonic output power, an ablation progress may be correspondingly determined by detecting a change of the pressure inside the balloon, and the end point of the ablation may be accurately determined by combining changes in the ablation area temperature and the outer surface impedance of the balloon.

It is to be noted that the ablation duration set by the control system is a total duration after the ultrasonic transducer starts to work. Another system parameter associated with the ablation duration is a sustained duration, which refers to the time period during which the ultrasonic transducer continuously works after the detected ablation area temperature reaches a set value. Both the ablation duration and the sustained duration are important parameters for automatically determining the end point of the ablation.

In S2 to S4, before the ultrasonic transducer starts to work, the balloon on an ablation catheter needs to be moved to a specified position in the target ablation area, and then the normal saline is injected into the balloon. The control system controls the normal saline to circulate for cooling. After the pressure inside the balloon reaches a set target value, outflow and inflow velocities are maintained consistent. Then, the pressure inside the balloon and the outer surface impedance of the balloon are started to be detected in real time, and are fed back to the control system. Because the pressure inside the balloon fluctuates up and down with a human pulse, the pressure inside the balloon uses an average value of a plurality of peak pressure values and trough pressure values before a moment as a pressure at the moment.

In an embodiment of the present disclosure, working modes of the ultrasonic transducer include two modes: an ablation mode and a temperature measurement mode. The ultrasonic transducer outputs relatively high power when working in the ablation mode, and outputs relatively low power when working in the temperature measurement mode.

In S5 to S6, after the pressure inside the balloon reaches the set target value, the ultrasonic transducer first enables the ablation mode to output ultrasonic energy to perform ablation on nerve tissue in the target ablation area. Meanwhile, the control system starts to calculate the ablation duration. After a particular ablation time, the ultrasonic transducer switches to the temperature measurement mode to detect the ablation area temperature, and feeds back the temperature to the control system. The control system compares the actually detected ablation area temperature with the set value of the ablation area temperature. When the actual value reaches the set value, the control system starts to calculate the sustained duration. Meanwhile, the control system performs a next working step.

The ablation area temperature is detected by an ultrasonic backscatter energy temperature measurement method. An ultrasonic wave will be scattered in tissue when passing through the tissue. Scattering intensity and direction are related to a temperature of the tissue. Therefore, the temperature of the tissue in the ablation area may be obtained by measuring intensity and a change of backscattering. Specifically, the ultrasonic transducer transmits a low-frequency ultrasonic wave (2.0 MHz to 4.0 MHz) and receives an echo signal. The echo signal is filtered to eliminate noise and then gain amplification is performed, a signal after a peak of a first echo is extracted, an amplitude is calculated (the first echo indicates a blood vessel wall), and a scattering coefficient of the tissue is calculated by using the amplitude of a backscattered signal.

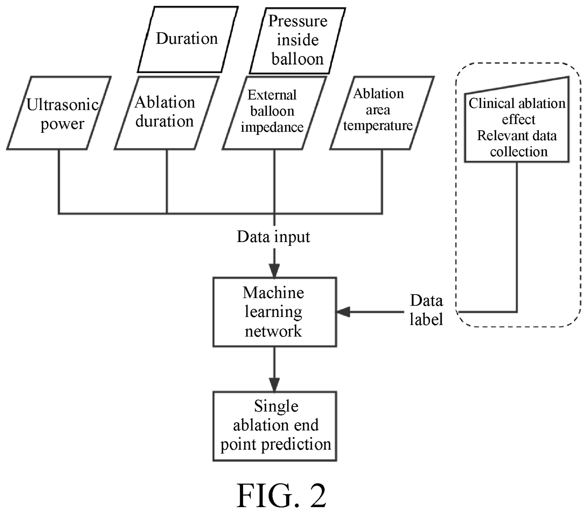

In an embodiment of the present disclosure, the determination of the end point of the ablation in S7 is to invoke a pre-trained machine learning model for determination. The pre-trained machine learning model is shown in FIG. 2. The pre-trained machine learning model automatically performs ablation effect evaluation and end point determination according to related working parameters input by the control system. Determination of reaching the end point of the ablation includes two cases: an ablation effect reaches an expected target and an abnormal interruption occurs.

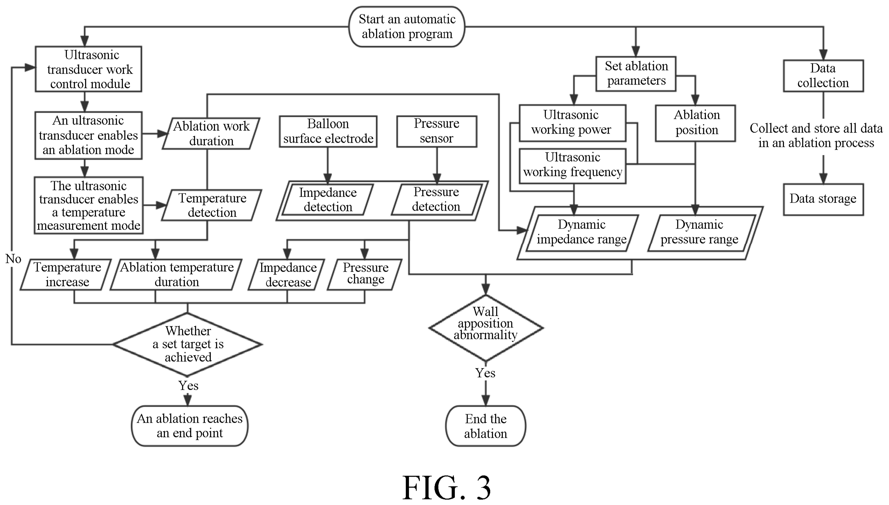

The machine learning model is obtained by establishing a data set through relevant data collected in a dose-effect relationship research experiment, a clinic experiment, and an animal experiment, and performing model training and adjustment test in advance. As shown in FIG. 3, specific effects of input related working parameters on effect evaluation and end point determination and relevant processes are described below in detail.

A case in which the ablation effect reaches the expected target and the end point of the ablation is reached needs to be jointly determined according to a plurality of working parameters and changes of the plurality of working parameters. The plurality of working parameters includes an ultrasonic output power, an ablation duration, a sustained duration, an ablation area temperature, a pressure inside a balloon, and an outer surface impedance of the balloon. A system dynamically adjusts an impedance range and a pressure range according to a set ultrasonic output power, working frequency, ablation position, and ablation duration, and calculates an impedance decrease value, a pressure change value, a temperature increase value, and the like. A machine learning model comprehensively determines, according to set values, measured values, and variations of the above plurality of working parameters, whether ablation work in the area reaches an end point of an ablation.

A case in which the end point of the ablation is reached due to the abnormal interruption include several cases in which parameters such as the ablation duration, the pressure inside the balloon, and the outer surface impedance of the balloon change abnormally.

The ablation duration set by the control system is a total duration after the ultrasonic transducer starts to work, and the set ablation duration is a maximum value. In an ablation process, an excessively long time for the balloon to inflate the blood in a blood vessel may cause blood to be unable to circulate for a long time, which may subsequently lead to ischemic necrosis of local tissue. Therefore, when a set ablation duration is exceeded, the ablation work in the current ablation area needs to be stopped even if an ablation effect is not achieved.

A state of tissue and an ablation effect in a nerve ablation process can be monitored by detecting an outer surface impedance value of the balloon in real time. In the ablation process, as a nerve is damaged by a high temperature, the impedance value gradually decreases. When a change of the outer surface impedance of the balloon abruptly changes abnormally, it indicates that a problem may occur in the ablation process. For example, the balloon may be detached from the blood vessel wall or another abnormality may occur. In this case, the ablation work in the current ablation area needs to be stopped even if an ablation effect is not reached.

An apposition condition between the balloon and the blood vessel wall and whether the blood vessel wall deforms seriously can be monitored by detecting a value of the pressure inside the balloon in real time. When the detected value of the pressure inside the balloon abruptly changes abnormally beyond the set value, it indicates that the blood vessel wall may deform seriously unexpectedly. In this case, the ablation work in the current ablation area needs to be stopped even if an ablation effect is not reached.

When it is determined that the ablation effect reaches an expected target and/or an abnormal interruption occurs to reach an end point of an ablation in the area, determining whether the current ablation work is completed is performed. If ablation work in another area further needs to be performed, S2 is performed; and S2 to S9 are repeatedly performed to perform the ablation work in another specified area. If the ablation work in another area does not need to be performed, the control system ends the work.

When it is determined that the end point of the ablation in the area is reached, S8 is performed, and the control system adjusts a value of output power of the ultrasonic transducer according to related working parameters such as the ablation area temperature detected in real time. S5 is performed, the ultrasonic transducer enables an ablation mode, and S5 to S8 are repeatedly performed to perform ablation work on the area again until the end point of the ablation is reached.

When the machine learning model determines that a single ablation reaches the end point of the ablation of the area and the current ablation work is ended, the control system records the working parameters of the current ablation process and an ablation result, and accumulates the working parameters for determining an end point of all ablation work. All data after a surgery are encrypted and saved for performing iterative update on the machine learning model with reference to a final ablation effect, as shown by a dashed line box in FIG. 2.

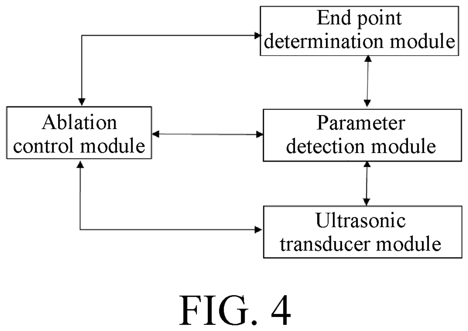

Based on the above automatic determination method for an intravascular nerve ablation end point, an embodiment of the present disclosure further provides an automatic determination method for an intravascular nerve ablation end point. As shown in FIG. 4, the automatic determination system includes an ablation control module, a parameter detection module, an ultrasonic transducer module, and an end point determination module.

The ablation control module is installed with an automatic ablation control program, and is configured to control parameter configuration for starting of ablation work and monitor an overall ablation working process. When the ablation work is controlled to start, specific working parameters are first configured and set according to different ablation areas and different ablation objectives. The working parameters at least include, but are not limited to, an ablation area, an ultrasonic working frequency, an ultrasonic output power, a pressure inside a balloon, an ablation duration, and an ablation area temperature. Secondly, after the balloon with a built-in ultrasonic transducer is moved to a specified position in a target ablation area, a circulating system is controlled to inject normal saline to the balloon until the pressure inside the balloon reaches a set target value. In addition, in an ablation working process, feedback information of the modules is received, related working parameters are adjusted, and related working parameters and variations of the related working parameters are input to the end point determination module, so as to evaluate and determine an end point of an ablation.

The parameter detection module is configured to detect actual values of the related working parameters in real time in the ablation working process, and feed back the actual values to the ablation control module and the end point determination module. The detected related working parameters include the pressure inside the balloon, an outer surface impedance of the balloon, the ablation area temperature, and the like.

The ultrasonic transducer module is configured to output ultrasonic energy to perform ablation treatment on nerve tissue, where working modes of the ultrasonic transducer module include an ablation mode and a temperature measurement mode. The ultrasonic transducer module outputs relatively high power when working in the ablation mode, and outputs relatively low power when working in the temperature measurement mode. In addition, the ablation control module may adjust a value of the ultrasonic output power in the ablation process according to the related working parameters such as the ablation area temperature detected in real time, to achieve a target ablation effect.

The end point determination module is based on a pre-trained machine learning model, and is configured to automatically evaluate and determine an end point of an ablation. Determination of reaching the end point of the ablation includes two cases: an ablation effect reaches an expected target and an abnormal interruption occurs. The end point determination module determines that an abnormal interruption occurs and the end point of the ablation is reached when a set ablation duration is reached, or parameters such as the pressure inside the balloon and/or the outer surface impedance of the balloon changes abnormally.

Determining that the ablation effect reaches the expected target and the end point of the ablation is reached needs to be jointly determined according to a plurality of working parameters and changes of the plurality of working parameters. Specifically, the end point determination module is based on a machine learning model and determines according to the plurality of input working parameters and variations of the input working parameters. The plurality of working parameters include an ultrasonic output power, an ablation duration, a sustained duration, an ablation area temperature, a pressure inside a balloon, an outer surface impedance of the balloon, and the like.

It is to be noted that the control system in this embodiment of the present disclosure may be implemented by a microprocessor or a single-chip computer in which an automatic ablation control program is installed. In some embodiments, the control system may alternatively be used as the above ablation control module.

In conclusion, compared with an existing technology, according to the automatic determination method for an intravascular nerve ablation end point in the embodiments of the present disclosure, high-accuracy automatic determination of the end point of the intravascular nerve ablation is achieved by setting and detecting a plurality of working parameters in real time and combining intelligent evaluation of the machine learning model. The method effectively avoids a false determination caused by determining relying on a single parameter, ensures safety in an ablation process, and optimizes an ablation effect. In addition, the method can adjust the ultrasonic output power according to real-time feedback, achieve the target ablation effect, and record data of each ablation to iteratively optimize the machine learning model, thereby continuously improving accuracy of determining the end point of the intravascular nerve ablation.

It is to be noted that the above plurality of embodiments are merely examples for description. Technical solutions of the embodiments may be combined, and all fall within the protection scope of the present disclosure.

In addition, terms "first" and "second" are merely used for the purpose of description, and cannot be construed as indicating or implying relative importance or implying a quantity of indicated technical features. Therefore, features defined by "first" and "second" may explicitly or implicitly include one or more of the features. In the description of the present disclosure, "a plurality of" means two or more, unless otherwise limited explicitly and specifically.

The foregoing describes an automatic determination method and system for an intravascular nerve ablation end point provided in the present disclosure in detail. Any obvious change made by a person of ordinary skill in the art to the present disclosure without departing from the essence of the present disclosure will constitute a violation of the patent right of the present disclosure and take corresponding legal responsibility.

## Claims

1. An automatic determination method for an intravascular nerve ablation end point, comprising the following steps:
(1) setting working parameters, wherein the working parameters at least comprise an ablation area, an ultrasonic working frequency, an ultrasonic output power, a pressure inside a balloon, an ablation duration, and an ablation area temperature;
(2) moving the balloon with a built-in ultrasonic transducer to a specified position in a target ablation area;
(3) injecting normal saline into the balloon, and maintaining consistent outflow and inflow velocities until the pressure inside the balloon reaches a set target value;
(4) detecting the pressure inside the balloon and an outer surface impedance of the balloon in real time, and feeding back the pressure and the outer surface impedance to a control system;
(5) enabling, by the ultrasonic transducer, an ablation mode to start ablation work, and starting, by the control system, to calculate the ablation duration;
(6) switching the ultrasonic transducer to a temperature measurement mode to detect the ablation area temperature, and feeding back the temperature to the control system; starting, by the control system, to calculate a sustained duration in a case that an ablation area temperature set value is reached;
(7) determining, by the control system according to related working parameters detected in real time, whether an end point of an ablation in the area is reached; performing step (9) in a case that the end point of the ablation in the area is reached; performing step (8) in a case that the end point of the ablation in the area is not reached;
(8) performing step (5) after the control system adjusts a value of the ultrasonic output power according to the ablation area temperature and the related working parameters detected in real time, and repeatedly performing steps (5) to (8);
(9) determining whether current ablation work is completed; performing step (10) in a case that the current ablation work is completed; performing step (2), and repeatedly performing steps (2) to (9) in a case that the current ablation work is not completed; and
(10) ending.

2. The automatic determination method for an intravascular nerve ablation end point according to claim 1, wherein
working modes of the ultrasonic transducer comprise an ablation mode and a temperature measurement mode, wherein the ultrasonic transducer outputs relatively high power when working in the ablation mode, and outputs relatively low power when working in the temperature measurement mode.

3. The automatic determination method for an intravascular nerve ablation end point according to claim 1, wherein
the end point of the ablation in the area is determined by using a machine learning model, and the machine learning model automatically performs ablation effect evaluation and end point determination according to the related working parameters input by the control system, wherein determination of reaching the end point of the ablation comprises two cases: an ablation effect reaches an expected target and an abnormal interruption occurs.

4. The automatic determination method for an intravascular nerve ablation end point according to claim 3, wherein
the related working parameters input by the machine learning model at least comprise set values, measured values, and variations of the ultrasonic output power, the ablation duration, the sustained duration, the ablation area temperature, the pressure inside the balloon, and the outer surface impedance of the balloon.

5. The automatic determination method for an intravascular nerve ablation end point according to claim 3, wherein
in an ablation working process, the machine learning model determines that the abnormal interruption occurs and stops ablation work in a current ablation area to reach the end point of the ablation when at least one of the following cases occurs: the ablation duration exceeds a set value, a change of the outer surface impedance of the balloon abruptly changes abnormally, or a value of the pressure inside the balloon exceeds a set value and abruptly changes abnormally.

6. The automatic determination method for an intravascular nerve ablation end point according to claim 1, wherein
in an ablation working process, the control system adjusts a value of output power of the ultrasonic transducer in a range below a set value of the ultrasonic output power according to the ablation area temperature and the related working parameters detected in real time, to achieve a target ablation effect.

7. An automatic determination system for an intravascular nerve ablation end point, comprising an ablation control module, a parameter detection module, an ultrasonic transducer module, and an end point determination module, wherein
the ablation control module is configured to: configure working parameters for starting ablation work, wherein the working parameters at least comprise an ablation area, an ultrasonic working frequency, an ultrasonic output power, a pressure inside a balloon, an ablation duration, and an ablation area temperature; perform comprehensive monitoring on an ablation working process;
the parameter detection module is configured to detect actual values of related working parameters in real time in the ablation working process, and feed back the actual values to the ablation control module and the end point determination module, wherein the related working parameters at least comprise the pressure inside the balloon, an outer surface impedance of the balloon, and the ablation area temperature;
the ultrasonic transducer module is configured to: output ultrasonic energy to perform ablation treatment on nerve tissue, wherein working modes of the ultrasonic transducer module comprise an ablation mode and a temperature measurement mode; adjust the ultrasonic output power according to the ablation area temperature and the related working parameters detected in real time in the ablation working process, to achieve a target ablation effect; and
the end point determination module is based on a pre-trained machine learning model, and is configured to automatically evaluate and determine an end point of an ablation according to a plurality of input working parameters and variations of the plurality of input working parameters.

8. The automatic determination system for an intravascular nerve ablation end point according to claim 7, wherein
the end point determination module determines that an abnormal interruption occurs and stops ablation work in a current ablation area to reach the end point of the ablation when at least one of the following cases occurs: the ablation duration exceeds a set value, a change of the outer surface impedance of the balloon abruptly changes abnormally, or a value of the pressure inside the balloon exceeds a set value and abruptly changes abnormally.
